# EUROPEAN PATENT APPLICATION

(11) **EP 4 575 504 A1**
(43) Date of publication of application: **25.06.2025**
(21) Application number: 24221036.7
(22) Date of filing: 18.12.2024
(51) Int. Cl.: G01N 33/68, G01N 33/542

(54) **MEANS AND METHODS FOR QUANTIFYING OR SCREENING FOR COMPLEX FORMATION**

(30) Priority: 18.12.2023 EP 23217720
(71) Applicant: NanoTemper Technologies GmbH, 81379 München (DE)
(72) Inventor: BAASKE, Philipp, 81369 Munich (DE); ADAMS, Nathan, 81369 Munich (DE)
(74) Representative: Schiweck Weinzierl Koch Patentanwälte Partnerschaft mbB

(57) **Abstract**

The present invention provides methods for quantifying and/or screening for the formation of complexes, in which two of the three compounds of the complex are proteins, one of the two tagged with a fluorescent label and the other of the two tagged with a fluorescence quencher. The third compound is a compound capable of linking those two proteins together, thus forming a ternary complex. The methods of the present invention consist of analysing the changes in fluorescence spectrum that occur following the formation of a complex comprising said compounds, due to the interaction between fluorescent label and fluorescence quencher that formation of said complex brings.

## Description

### I. FIELD OF THE INVENTION

The present invention relates to a method for quantifying the formation of a complex composed of a first, second, and third compound, wherein said second compound is capable of attaching to said first and third compound, comprising equipping said first or third compound with a fluorescent quencher, while the other one of the two is equipped with a fluorescent label. Such a complex may be described as a ternary complex.

### II. BACKGROUND

A ternary complex is a protein complex containing three molecules that are bound together. Examples are a crystal containing a protein with two small molecules bound, for example, cofactor and substrate; or a complex formed between two proteins and a single substrate. In immunology, a ternary complex may be seen in a MHC-peptide-T-cell-receptor complex.

A further ternary complex may be formed by a bispecific antibody directed against two different targets. Namely, each target together is a compound of the ternary complex and the bispecific antibody is the third compound.

Ternary complexes are also formed by proteolysis targeting chimeras (PROTACs). A PROTAC, first described in 2001 (Sakamoto et al., doi: 10.1073/pnas.141230798), is a heterobifunctional molecule composed of two active domains and a linker, capable of removing specific unwanted proteins. Rather than acting as a conventional enzyme inhibitor, a PROTAC works by inducing selective intracellular proteolysis. Specifically, the so-called warhead of a PROTAC binds a to-be-degraded target, while the other part of a PROTAC binds a member of the ubiquitin pathway, thereby effecting ubiquitination of the target and, thus, its degradation.

There are various challenges that prevent the accurate and unambiguous identification of the hook effect in assays designed to quantify ternary complex formation, thus hindering the development of medical treatments that rely on ternary complexes. The hook effect can be seen, for example, when analysing the binding properties of PROTACs, and may be described as a phenomenon wherein the ternary complex formation decreases with increasing concentration of the PROTAC molecules once PROTAC concentration crosses a certain peak point. This is due to higher concentrations of PROTACs favouring binary complex formation due to binding site saturation, rather than ternary complex formation, resulting in "hook"-shaped activity versus concentration curve. The hook effect is also observed in the assessment of binding properties of bispecific antibodies. Accurate and unambiguous detection of the concentration at which the hook effect begins to occur, and thus productive ternary complex formation peaks, is therefore critical to developing, e.g. novel PROTACs or bispecific antibodies.

Namely, Cecchini et al (2021; doi.org/10.3389/fchem.2021.672267) observed issues with the hook effect when working with PROTACs. Accordingly, Cecchini suggests increasing cooperative-binding protein-protein interaction to stabilize ternary complexes in order to reduce the hook effect. This restricts the rate at which PROTACs can be assessed for their ability to form ternary complexes and hence delays pre-clinical development of PROTACs.

As stated above, the hook effect also affects the development of bispecific antibodies for the same reasons, see Mak et al. (2023, doi: 10.1080/19420862.2022.2149053); Chen et al. (2021; doi.org/10.1080/19420862.2021.1914359).

In sum, research aiming to develop and optimize novel PROTACs, along with other biotherapeutics that function via ternary complex formation, such as bivalent antibodies, has been hindered by limitations in the performance of assays required to analyse ternary complex formation. These limitations include the inability to accurately and unambiguously identify the hook effect, limitations in throughput, and high sample consumption whilst performing said assays. Being able to perform high throughput, automated analysis of ternary complex formation via a method that can accurately identify the hook effect would massively accelerate development of PROTACs and bivalent antibodies by allowing for mass screening for PROTAC compounds that form high affinity ternary complexes that display positive binding cooperativity, thus enhancing their *in vivo* potency. There is thus a need to address these issues.

The technical problem underlying the present invention is therefore to address these needs and technical objectives.

The present invention addresses these needs and technical objectives and provides a solution as described herein, as defined in the claims as well as exemplified in the appended examples and illustrated in the figures.

Generally speaking, the present invention provides methods for quantifying and/or screening for the formation of ternary complexes, in which two of the three compounds of the complex are proteins, one of the two tagged with a fluorescent label and the other of the two tagged with a fluorescence quencher. The further compound completing the ternary complex is a compound capable of linking those two proteins together, thus forming a ternary complex. The methods of the present invention comprise analysing the changes in fluorescence spectrum that occur following the formation of a ternary complex comprising said compounds, due to the interaction between fluorescent label and fluorescence quencher that formation of said ternary complex brings.

### III. DETAILED DESCRIPTION

Accordingly, the present invention provides a method for quantifying the formation of a complex composed of a first, second and third compound, wherein said second compound is capable of attaching to said first and third compound, wherein said first or third compound is equipped with a fluorescence quencher, while the other one of the two, i.e. the other one of the first or third compound, is equipped with a fluorescent label. Such a complex may be described as a ternary complex. The present invention also provides for a method for quantifying the formation of a complex composed of a first, second and third compound, wherein said second compound is capable of attaching to said first and third compound, wherein said first or third compound is equipped with a fluorescence quencher, while the other one of the first and third compound is equipped with a fluorescent label, comprising generating a concentration-response curve that shows the peak of ternary complex formation, whereby said peak corresponds to the lowest level of fluorescence emission intensity, and determining whether subsequent to said lowest level of fluorescence emission intensity of fluorescence increases in said concentration-response curve, which is indicative of a hook effect, thereby quantifying ternary complex formation.

When used in the present invention, "quantifying the formation of a complex" refers to the quantitative assaying of the relationship between concentration of compound present (or another relevant factor) and the amount of complex, either relative or absolute, that is formed.

The present invention also provides a method for screening whether a second compound is capable of attaching to a first and third compound to form a complex, comprising detecting whether a complex is formed between said first, second and third compounds, wherein said first or third compound is equipped with a fluorescence quencher, while the other one of the two, i.e. the other one of the first or third compound, is equipped with a fluorescent label. The present invention also provides for a method a method for screening whether a second compound is capable of attaching to a first and third compound to form a complex, comprising detecting whether a complex is formed between said first, second and third compound, wherein said first or third compound is equipped with a fluorescence quencher, while the other one of the first and third compound is equipped with a fluorescent label, comprising generating a concentration-response curve that shows the peak of ternary complex formation, whereby said peak corresponds to the lowest level of fluorescence emission intensity, and determining whether subsequent to said lowest level of fluorescence emission intensity of fluorescence increases in said concentration-response curve, which is indicative of a hook effect, thereby screening a second compound capable of attaching to the first and third compound.

The present invention further relates to a method wherein said first and third compounds are proteins and wherein said second compound is a proteolysis-targeting chimera (PROTAC).

The present invention further relates to a method wherein said second compound is a compound with a molecular weight less than 2000 Da.

The present invention further relates to a method wherein said second compound is a bifunctional polypeptide, wherein said bifunctional polypeptide is preferably a bispecific antibody.

The present invention further relates to a method wherein quantifying or screening for the formation of a ternary complex comprises analyzing alterations in the fluorescence spectrum that occur upon ternary complex formation between said first, second and third compound, wherein preferably alterations in the fluorescence spectrum alterations are alterations to the fluorescence emission spectrum, wherein more preferably an alteration in the fluorescence emission spectrum is indicative of ternary complex formation. In some embodiments, alterations in the fluorescence spectrum are analyzed by microscale thermophoresis, temperature-related intensity charge or spectral shift technology

The present invention further relates to a method wherein said second compound is absent.

The present invention further relates to the use of a fluorescence quencher for quantifying ternary complex formation of a first, second and third compound, wherein said second compound is capable of attaching to said first and third compound, wherein said first or third compound is equipped with a fluorescence quencher, while the other one of the two, i.e. the other one of the first or third compound, is equipped with a fluorescent label.

The aforementioned methods can be performed via the use of a kit and can be further defined by a system comprising the aforementioned aspects of the methods described.

Much to their surprise, the present inventors found that by using a fluorescence quencher, they were able to accurately and unambiguously detect and quantify the hook effect. By quantifying ternary complex formation via the proximity-induced quenching of a fluorophore that occurs only when all three members are bound in said ternary complex, a concentration-response curve can be generated that shows the peak of ternary complex formation, corresponding to the lowest level of fluorescence emission intensity, and subsequently the decrease in ternary complex formation, which corresponds to a subsequent increase in fluorescence as proximity-induced quenching is reduced by dissociation of the ternary complex in favour of binary complexes (i.e. the hook effect).

As described in the prior art and used herein, the hook effect refers to the observation that if a multivalent compound acts as a linker between two parts of a ternary complex, then increasing the amount of linker compound in the mixture does not always increase the amount of ternary complex formed. On the contrary, at higher concentration ranges, the amount of ternary formed complex may actually decrease. This is due to the increased availability of free linker resulting in an increased formation of binary complexes, rather than the intended ternary complex. Ternary complex formation requires the initial formation of a binary complex between the one of the ligand domains of the multivalent compound and one of the two proteins. This binary complex must then bind to an unoccupied binding site of the other of the two proteins via the second ligand domain of the multivalent compound. If said binding site is occupied by another linker, this prevents formation of a ternary complex.

At lower concentrations of linker, target protein (P), linker (L), and ubiquitin pathway protein (E) may proceed from monomers to ternary complex (PLE) via intermediate binary complexes PL and EL: 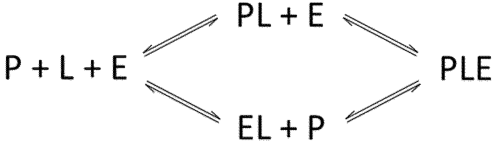 At higher concentrations of linker compound, rate of ternary complex formation slows in favour of formation of inactive heterodimers, PL and EL:

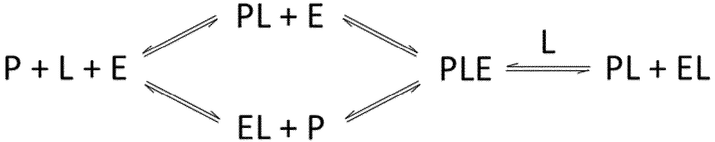

The hook effect is commonly seen when employing assays to quantify the amount of an analyte present in a sample via a method requiring ternary complex formation. For example, the use of one-step immunoassays that utilize both a capture and detection antibody to quantify the presence of an antigen. Formation of a ternary complex consisting of both antibodies and the antigen of interest will be impaired by very high antigen concentration as a result of the hook effect, thus resulting in an underestimate of the true concentration of antigen. The same is true for quantification of ternary complexes when developing novel PROTACs, which can form unproductive binary complexes that do not induce degradation of the target protein of interest. It is therefore important to be able to identify the concentration of PROTAC at which the hook effect begins to occur. An inability to accurately quantify the formation of a ternary complex and assess the extent of the hook effect may hinder the development of novel PROTAC compounds.

Demonstration of hook effect-induced dissociation via Spectral Shift assays is possible, but relies on binary and ternary complexes having significantly different signals, which is not guaranteed. Whilst it may be possible to identify a change in Kd, indicative of cooperative binding, Spectral Shift assays may also fail to reveal a change in ratio i.e. the "hook" signal. It is important to be able to accurately identify the concentration of PROTAC compound at which the hook effect begins to occur, as being able to identify that point means that modifications to PROTAC structure can be made to enhance positive binding cooperativity and hence allow for an increased concentration of PROTAC whilst maintaining productive PROTAC ternary complex formation. This is because an inability to accurately identify the concentration at which the hook effect may begin to occur prevents being able to quantitatively demonstrate whether or not a modification increases or decreases that concentration. Improving the positive binding cooperativity of the ternary complex will prevent the hook effect from occurring until higher PROTAC concentrations are reached. Binding cooperativity occurs when a molecule has multiple ligand binding sites and the binding of a first ligand alters the binding affinity of subsequent ligands when they bind to the same molecule at another site. Positive binding cooperativity refers to an enhancement of binding affinity, whereas negative binding cooperativity refers to reduced binding affinity. When considering the formation of ternary complexes, positive binding cooperativity results in increased stability of said ternary complex and as discussed, thereby enhances ternary complex formation over binary complex formation.

Other biophysical methods of quantifying the hook effect include FRET (Förster Resonance Energy Transfer) but FRET lacks significant controls and is not as readily interpreted as the method of the present invention. Surface based techniques, including but not limited to surface plasmon resonance (SPR), claim to be able to quantify ternary complex formation, but face challenges in performance due to biochemical artefacts that are likely to be introduced due to avidity.

The methods of the present invention may generally be applied in connection with known methods suitable to measure alterations in the fluorescence spectrum, e.g., those being based on measuring fluorescence emission relying on the Stoke shift. For example, in one embodiment of the present invention, the alterations in the fluorescence spectrum of said labelled protein comprising an Fc portion (e.g., antibody) may be analysed by microscale thermophoresis, temperature-related intensity change or spectral shift technology as described in WO 2008/061706, WO 2018/234557, and WO 2023/275274, respectively.

As used herein, "ternary complex" refers to a complex that results from the binding together of three independent molecules, either permanently or reversibly. Said molecules may comprise three of the same compound or may comprise two or three different compounds. "Complex", as used herein, refers to a molecular entity formed by loose association involving two or more component molecular entities (ionic or uncharged), or the corresponding chemical species.

The methods of the present invention comprises equipping said first or third compound with a fluorescence quencher, while the other one of the two is equipped with a fluorescent label.

The method of the present invention can be used to quantify ternary complexes that form in presence of a compound that is capable of simultaneously binding to both of the other two compounds of the complex, thus forming a ternary complex.

In some embodiments, this will be a PROTAC.

In the methods of the present invention, this will result in proximity-induced interaction between the fluorescence quencher and fluorescent label of the other two compounds. The methods of the present invention therefore rely on analysing alterations in the fluorescence spectrum of the fluorophore that result from that interaction. This method allows for high-throughput, automatable analysis that can readily be used to identify key parameters associated with ternary complex formation and the concentration of reagent that leads to peak ternary complex formation. This is displayed below in **Example 1.** The methods of the present invention thus allow for definitive demonstration of ternary complex formation that does not rely on distinguishing between what may be small differences in fluorescence emission spectra between binary and ternary complexes.

In one embodiment of the present invention, the first and third compounds of the ternary complex are proteins.

Proteins (including fragments thereof, preferably biologically active fragments, and peptides, usually having less than 30 amino acids) comprise one or more amino acids coupled to each other via a covalent peptide bond (resulting in a chain of amino acids). The term "peptide" as used herein describes a group of molecules which typically comprise more than 2 amino acids and usually not more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a hetero-multimer is an antibody molecule, which, in its naturally occurring form, consists of two identical light polypeptide chains and two identical heavy polypeptide chains. The terms "peptide" and "protein" also refer to naturally modified polypeptides/proteins wherein the modification is effected e.g. by post-translational modifications like glycosylation, acetylation, phosphorylation and the like. Such modifications are well known in the art. A preferred example of a peptide as described above is a Fc-binding peptide.

In one embodiment of the present invention, said second compound comprises (i) a target protein binding moiety that is capable of attaching to a target protein which is a first compound as defined in any one of the preceding claims and (ii) an ubiquitin pathway protein binding moiety that is capable of attaching to a ubiquitin pathway protein which is a third compound as defined in any one of the preceding claims, and (iii) a linker moiety that covalently couples them. In a further embodiment of the present invention, said ubiquitin pathway protein binding moiety is a ligand for a component of an ubiquitin protein ligase complex, wherein said ubiquitin pathway protein is preferably a component of an ubiquitin protein ligase complex, wherein said component of an ubiquitin protein ligase complex is more preferably an ubiquitin protein ligase. In some embodiments of the invention, the component of the ubiquitin protein ligase complex effects ubiquitination of a (the) target protein. In a specific embodiment of the present invention, said second compound is a proteolysis targeting chimera (PROTAC).

PROTACs are compounds capable of mediating targeted protein degradation (TPD) by inducing interactions between a protein-of-interest (POI) and a suitable ubiquitin pathway protein (UPP). This is achieved by the PROTAC possessing two independent ligand domains, one capable of binding to said POI and one capable of binding to said component of the ubiquitin pathway. Simultaneous interaction between the two ligand domains and their respective targets brings the POI and component of the ubiquitin pathway into sufficient proximity to induce ubiquitination of the POI, thus resulting in degradation via endogenous protein degeneration pathways. The use of an artificial compound that induces ubiquitination through bringing a target protein into sufficient proximity with a ubiquitin pathway protein was demonstrated in WO02/20740A2. Alternative methods to induce ubiquitination include providing a ubiquitin protein ligase polypeptide that encodes a ubiquitin conjugation activity, thereby functionally linking the ubiquitin protein ligase polypeptide to a target polypeptide interaction domain that provides a target polypeptide recruitment activity, and then expressing the ubiquitin protein ligase polypeptide-target polypeptide interaction domain in a cell, as described in WO00/22110 and WO02/020740.

As used herein, component of the ubiquitin pathway refers to any protein capable of mediating, directly or indirectly, ubiquitination and thereby inducing the subsequent proteasomal degradation of a target protein. This includes, but is not limited to, E3 ubiquitin ligases such as cereblon (CRBN), Von Hippel-Lindau (VHL), Mouse double minute 2 homolog (MDM2), and cellular Inhibitor of Apoptosis Protein 1 (clAP1).

PROTACs may be used to target proteins that are "undruggable" by means of conventional small molecular inhibitors. "Undruggable", as used herein, refers to biological molecules "whose functional interfaces are flat and lack defined pockets for ligand interaction" (Xie et al., 2023; doi:10.1371/journal.pcbi.1010974), thus rendering the design of conventional small molecule occupancy-based drugs highly challenging. PROTACs act not via occupancy-based pharmacology, but by "event-driven pharmacology", reducing the activity of a given protein by inducing its permanent degradation rather than occupancy of its functional domain. Thus, PROTACs may be used to treat diseases characterized by dysfunctional over activity and/or overexpression of a protein.

PROTAC function requires the combination of three distinct moieties - a ligand domain capable of binding to the POI, a ligand domain capable of binding to the ubiquitin pathway protein, and a linker domain that links the two ligand domains and thus the two proteins once the ternary complex has been formed. Ligand domains typically interact non-covalently with their respective targets and can therefore function "enzymatically", to bind and induce the proteasomal degradation of multiple copies of the target POI via sequential cycles of binding, induction of ubiquitination, and then dissociation of the complex to allow the PROTAC to bind a novel POI. As PROTACs are envisaged to allow the targeting of currently "undruggable" targets, identification of suitable candidate molecules to act as ligands for such targets can prove challenging, as there may be few or no example molecules on which to base potential candidate ligands. As with the POI ligand, care must also be taken when selecting the ubiquitin ligase ligand so as to ensure successful protein knockdown. This means selecting a ligand that will bind to an appropriate E3 ubiquitin ligase, or other member of a ubiquitin pathway. This is in part due to the tissue-selective expression of different E3 ubiquitin ligases, meaning that an appropriate ligase must be targeted if the POI is also expressed, or must be targeted, in a tissue-selective manner. Genomic analysis predicts the existence of over 600 E3 ubiquitin ligases in the human genome.

PROTACs must possess a linker domain to connect the two ligand domains in order to bring the POI and UPP into sufficient proximity for ubiquitination of the POI to occur. "Linker domain", as used herein and in the context of the present invention, refers to the moiety of the second compound that does not directly participate in binding, but instead joins the two ligand binding domains together. The linker domain must have a suitable geometric and chemical composition such that the POI and the UPP are brought together in a manner that allows for favourable protein-protein interactions and ubiquitination to occur. Polyethylene glycol (PEG) chain linkers are the most common PROTAC linkers employed thus far, owing to their flexibility and polar nature, but rigidifying the linker via heterocyclic scaffolding has been shown to improve stability of ternary complex formation and thereby enhance protein degradation in certain PROTACs. It has also been observed that the composition of the linker domain can influence PROTAC ligand-binding selectivity, with linker chain length demonstrated to alter POI targeting selectivity (Burslem et al., 2018, doi: 10.1016/j.chembiol.2017.09.009; Nowak et al., 2018, doi: 10.1038/s41589-018-0055-y).

Kinetics of ternary complex formation are influenced by both protein-protein and protein-small molecule interactions and are highly challenging to effectively model and predict. Small changes to the structure of any one of the components of a ternary complex can alter overall affinity and efficiency of binding, and the positive or negative cooperativity of ternary complex formation that results from the interaction between the two ligand domains of the small molecule is also greatly affected by structural changes to the ligand domains and linker domain (B. Han, 2020, doi: 10.1074/jbc.RA120.014715). This, along with other factors, contributes to the difficulty in rational PROTAC design that means that the dominant strategy in developing novel PROTACs has been the manual *in vitro* screening of many variants to identify optimal design (Bai, N. et al., 2021, doi: 10.1021/acs.jcim.0c01451).

By bringing a target protein and a ubiquitin pathway protein into close proximity, this second compound instigates the ubiquitin-mediated proteasomal degradation of the target protein. Ubiquitin-mediated proteasomal degradation refers to the proteolysis pathway in which proteins to be degraded are tagged with ubiquitin, a process termed "ubiquitination". This occurs via three main steps - activation of ubiquitin via an E1 ubiquitin-activating enzyme; conjugation of ubiquitin to an E2 ubiquitin conjugating enzyme; and finally ligation, in which an E3 ubiquitin ligase catalyses the transfer of one or more ubiquitin moieties to the protein. E3 ubiquitin ligases are a large family of enzymes that perform the same function i.e. catalysing the transfer of ubiquitin from an E2 ubiquitin conjugating enzyme to a target protein, but may exhibit low sequence homology. They may be divided into four groups based on structure and function: HECT type, U-box type, RING-finger type, and RBR type. In embodiments in which one of the three compounds present in the ternary complex is an E3 ubiquitin ligase, the methods of the present invention may be applied to any E3 ubiquitin ligase.

In some embodiments, the second compound is a bifunctional polypeptide, wherein said bifunctional polypeptide is preferably a bispecific antibody. As used herein, "bifunctional polypeptide" shall be used to refer to a polypeptide that can target and bind to two different compound simultaneously. As used herein, a bispecific antibody refers to an antibody or antibody fragment that is capable of binding to two different antigens, or two different epitopes present on the same antigen, simultaneously. Bispecific antibodies also exhibit the hook effect and similar issues in quantifying the formation of ternary complexes containing bispecific antibodies occur as described above.

Preferably, in the context of the present invention, if one of the compounds of the ternary complex is an antibody, in particular a bispecific antibody, said antibody, in particular, said bispecific antibody is not labelled with either one or two Fc binding peptides, wherein said Fc binding peptides have a fluorophore, blackhole quencher or FRET pairs.

An "antibody" when used herein is a protein comprising one or more polypeptides (comprising one or more binding domains, preferably antigen binding domains) substantially or partially encoded by immunoglobulin genes or fragments of immunoglobulin genes and comprises at least one Fc region or parts thereof as long as such parts are bound by said Fc-binding peptide. The term "immunoglobulin" (Ig) is used interchangeably with "antibody" herein. The recognized immunoglobulin genes include the kappa, lambda, alpha, gamma, delta, epsilon and mu constant region genes, as well as myriad immunoglobulin variable region genes. In particular, an "antibody" when used herein, is typically tetrameric glycosylated proteins composed of two light (L) chains of approximately 25 kDa each and two heavy (H) chains of approximately 50 kDa each. Two types of light chain, termed lambda and kappa, may be found in antibodies. Depending on the amino acid sequence of the constant domain of heavy chains, immunoglobulins can be assigned to five major classes: A, D, E, G, and M, and several of these may be further divided into subclasses (isotypes), e.g., IgG1, IgG2, IgG3, IgG4, lgA1, and IgA2. An IgM antibody consists of 5 of the basic heterotetramer unit along with an additional polypeptide called a J chain, and contains 10 antigen binding sites, while IgA antibodies comprise from 2-5 of the basic 4-chain units which can polymerize to form polyvalent assemblages in combination with the J chain. In the case of IgGs, the 4-chain unit is generally about 150,000 Daltons (Da).

Each light chain includes an N-terminal variable (V) domain (VL) and a constant (C) domain (CL). Each heavy chain includes an N-terminal V domain (VH), three or four C domains (CHs), and a hinge region. The constant domains are not involved directly in binding an antibody to an antigen.

When used herein the term "antibody" does not only refer to an immunoglobulin (or intact antibody), but also to a fragment thereof, and encompasses any polypeptide comprising an antigen-binding fragment or an antigen-binding domain which still contains at least one Fc region, e.g., scFv-Fc, IgNAR or hclgG fragments. Typically, such fragments would comprise an antigen-binding domain and have the same properties as the antibodies described herein.

The term "antibody" also includes but is not limited to polyclonal, monoclonal, monospecific, polyspecific such as bispecific, non-specific, humanized, human, single-chain, chimeric, synthetic, recombinant, hybrid, mutated, grafted, and in vitro generated antibodies, with a polyclonal antibody being preferred. Said term also includes domain antibodies (dAbs) and nanobodies. A "bispecific" or "bifunctional antibody" is an artificial hybrid antibody having two different heavy/light chain pairs and two different binding sites. Bispecific antibodies can be produced by a variety of methods including fusion of hybridomas or linking of binding fragments. See, e.g., Songsivilai & Lachmann, Clin Exp Immunol (1990), 79: 315-321; Kostelny et al., J Immunol (1992), 148: 1547-1553.The term "antibody" as used herein comprises antibodies as described herein, from any origin, e.g., human, mouse, rat, rabbit, goat, camel, llama, and others, and also includes in vitro-derived antibodies and modified antibodies such as humanized antibodies. "Humanized" forms of non-human (e.g., murine) antibodies are chimeric immunoglobulins, immunoglobulin chains or fragments thereof of mostly human sequences, which contain minimal sequence derived from non-human immunoglobulin. For the most part, humanized antibodies are human immunoglobulins (recipient antibody) in which residues from a hypervariable region (also CDR) of the recipient are replaced by residues from a hypervariable region of a non-human species (donor antibody) such as mouse, rat, rabbit, goat, camel, llama, and others, having the desired specificity, affinity, and capacity. Furthermore, "humanized antibodies" as used herein may also comprise residues which are found neither in the recipient antibody nor the donor antibody. These modifications are made to further refine and optimize antibody performance.

The humanized antibody as used herein also comprise at least a portion of an immunoglobulin constant region (Fc), typically that of a human immunoglobulin. For further details, see Jones et al., Nature (1986), 321: 522-525; Reichmann et al., Nature (1988), 332: 323-329; and Presta, Curr. Op. Struct Biol (1992), 2: 593-596. As used herein, "in vitro derived antibody" refers to an antibody where all or part of the variable region (e.g., at least one CDR) is generated in a non-immune cell selection (e.g., an in vitro phage display, protein chip or any other method in which candidate sequences can be tested for their ability to bind to an antigen). This term thus preferably excludes sequences generated by genomic rearrangement in an immune cell.

As used herein and unless specified otherwise, the term "antibody" also comprises chemoimmuno-conjugates, particularly antibody-drug-conjugates (ADC) as known to the skilled person, where, e.g., chemotherapeutic agents are linked to antibodies, e.g., cytostatic drugs or cytotoxic agents.

The term "bispecific" as used herein refers to an antibody construct which is "at least bispecific", i.e., it comprises at least a first binding domain and a second binding domain, wherein the first binding domain binds to one antigen or target, and the second binding domain binds to another antigen or target. The term "bispecific antibody construct" of the invention also encompasses multispecific antibody constructs such as trispecific antibody constructs, the latter ones including three binding domains, or constructs having more than three (e.g. four, five...) specificities.

Bispecific antibodies do not occur naturally and they are markedly different from naturally occurring products. A "bispecific" antibody is hence an artificial hybrid antibody or immunoglobulin having at least two distinct binding sites with different specificities. Bispecific antibody constructs can be produced by a variety of methods including fusion of hybridomas or linking of Fab' fragments.

The at least two binding domains and the variable domains of a bispecific antibody may or may not comprise peptide linkers (spacer peptides). The term "peptide linker" comprises in accordance with the present invention an amino acid sequence by which the amino acid sequences of one (variable and/or binding) domain and another (variable and/or binding) domain of the antibody construct of the invention are linked with each other. An essential technical feature of such peptide linker is that it does not comprise any polymerization activity.

As used herein, a "bispecific single chain antibody" denotes a single polypeptide chain comprising two binding domains. Each binding domain comprises one variable region from an antibody heavy chain ("VH region"), wherein the VH region of the first binding domain specifically binds to the CD3ε molecule, and the VH region of the second binding domain specifically binds to a cell surface antigen, as defined in more detail below. The two binding domains are optionally linked to one another by a short polypeptide spacer. A non-limiting example for a polypeptide spacer is Gly-Gly-Gly-Gly-Ser (G-G-G-G-S) and repeats thereof. Each binding domain may additionally comprise one variable region from an antibody light chain ("VL region"), the VH region and VL region within each of the first and second binding domains being linked to one another via a polypeptide linker, but in any case long enough to allow the VH region and VL region of the first binding domain and the VH region and VL region of the second binding domain to pair with one another such that, together, they are able to specifically bind to the respective first and second molecules.

One of the two compounds of the ternary complex as described herein comprises a fluorescent label. In one embodiment of the present invention, said fluorescent label may be a fluorescent dye. In accordance with the present invention, examples for such fluorescent dyes may comprise Cy5, Cy3, Atto647, Atto647N, Alexa647, Dy647, and variants thereof.

In the context of the present invention, the terms "label" and "dye" are used interchangeably and preferably refer to a fluorophore/fluorochrome, i.e., a fluorescent chemical compound, which is reemitting light upon excitation. Labels useful in the present invention comprise labels which are sensitive to environmental changes, i.e., the fluorescence spectrum of the dye alters upon environmental changes such as changes in the chemical microenvironment (ligand binding, conformational changes) and/or macroenvironment (e.g., location in LNPs versus location in cells) and/or temperature changes (e.g., heating or cooling).

Fluorescent labels for use according to the present invention can be selected from the group consisting of intrinsic fluorescent labels, fusion proteins, extrinsic fluorescent labels and the like. Intrinsic fluorescent labels include tryptophan residues, tyrosine residues, phenylalanine residues. Fusion proteins can be selected from the group consisting of blue-emitting fluorescent proteins, cyan-emitting fluorescent proteins, green-emitting fluorescent proteins, yellow emitting fluorescent proteins and red-emitting fluorescent proteins and the like. Reference is made to FPbase, a well-known data base in the art which provides a comprehensive list of presently known fluorescent proteins (https://www.fpbase.org/table/; Lambert, TJ (2019) FPbase: a community-editable fluorescent protein database. Nature Methods. 16, 277-278. doi:10.1038/s41592-019-0352-8).

In one embodiment of the present invention, the fluorescent labels may be extrinsic fluorescent labels. Extrinsic fluorescent labels can include but are not limited to commercially available labels, such as Cyanine dyes including Cy5, Cy3, Atto647, Atto647N, Alexa647 Dy647, and the like. Examples for extrinsic fluorescent labels are environment sensitive dyes described for example in WO 2018/234557, which is incorporated herein by reference. Environment sensitive dyes are known in the art and are described for example in Klymchenko, A. S. (2017) (Solvatochromic and fluorogenic dyes as environment-sensitive probes: design and biological applications. Accounts of Chemical Research, 50(2), 366-375.). In this context, WO 2018/234557 relates to fluorescent labels which are highly sensitive to environmental changes, e.g., changes in the chemical composition, temperature changes and the like.

In one embodiment of the present invention, the fluorescence quencher is a black hole quencher^{®}, for example BHQ1, BHQ2 or BHQ3.6.

In the context of the present invention, fluorescence quenching, as used herein, refers to any process that decreases the fluorescence intensity of a sample. This can occur via multiple molecular mechanisms - for example, absorption of excitation energy from a fluorophore and dissipation of that energy as non-light energy, such as heat. The present invention does not depend on the molecule mechanism by which quenching may occur. A fluorescence quencher, as used herein, therefore refers to any substance that can quench the fluorescence of a fluorophore. As such, when a fluorescence quencher comes within sufficient proximity of a fluorophore, fluorescence is quenched and a lower fluorescence signal is observed. In some embodiments of the present invention, the fluorescent quencher shall be a black hole quencher^{®} (BHQ) is a broad spectrum fluorescence quencher. Thus, when a BHQ interacts with a fluorescent label, the fluorescence emission intensity of said fluorescent label is reduced across a broad range of emission wavelengths. As used herein, "black hole quencher" may refer to any broad spectrum fluorescence quencher including, but not limited to, BHQ1, BHQ2 or BHQ3.6.

In one embodiment of the present invention, the method of the present invention comprises quantifying or screening for the formation of the ternary complex by analysing alterations in the fluorescence spectrum that occur upon ternary complex formation between said first, second and third compound, wherein preferably alterations in the fluorescence spectrum alterations in the fluorescence emission spectrum, wherein more preferably an alteration in the fluorescence spectrum is indicative of ternary complex formation.

Alterations in the fluorescence spectrum according to the present invention include changes in the fluorescence intensity of a fluorescent label, but also include spectral shifts and/or broadening or narrowing of their spectrum. According to the present invention, particularly where spectral shift technology is applied, it is preferred to detect the fluorescence at different wavelengths or wavelengths ranges, which could be achieved, e.g., by using bandpass filters. The detected intensities at these different wavelengths/wavelength ranges and the respective ratio allow a detection of a spectral shift of the entire emission spectrum, a broadening and/or narrowing of the spectrum. In this context, the spectral shifts preferably include bathochromic (i.e. red) shifts and/or hypsochromic (i.e. blue) shifts. The present invention relies on the use of fluorescence quenchers, attached to one of the three compounds of the ternary complex to be investigated. It is therefore preferred that quantification and/or screening for ternary complex formation via alterations in the fluorescence spectrum comprise detecting decreases in fluorescence emission intensity that may be proportionate to ternary complex formation.

In accordance with the present invention, fluorescence can be measured as known by the skilled person. According to the present invention, preferable means for exciting, preferably fluorescently exciting the labelled compound may be any suitable device selected from the group consisting of laser, fibre laser, diode-laser, light emitting diodes (LEDs), Halogen, LED-Array, HBO (HBO lamps are, e.g., short arc lamps in which the discharge arc fires in an atmosphere of mercury vapour under high pressure ), HXP (HXP lamps are, e.g., short arc lamps in which the discharge arc burns in an atmosphere of mercury vapour at very high pressure e.g., in contrast to HBO lamps they are operated at a substantially higher pressure and they employ halogen cycle. HXP lamps generate UV and visible light, including significant portion of red light) and the like. Preferably, in the context of the present invention the excitation light source enables a highly focused excitation. In the context of the present invention, the excitation light source is preferably a laser, even more preferably an LED.

It is understood by the person skilled in the art that the term "fluorescence" as employed herein is not limited to "fluorescence" per se but that the herein disclosed means, methods and devices may also be used and employed by usage of other means, in particular luminescence, such as phosphorescence. The person skilled in the art is aware that in the context of this invention the "excitation" wavelength and the "emission" wavelengths have to be separated.

Although the analysis of fluorescently labelled particles according to the present invention does not necessarily rely on temperature induced fluorescence intensity changes, the fluorescence intensity measurement may be performed at a constant predetermined temperature or during a defined temperature perturbation. Accordingly, in accordance with the present invention, it is possible to apply a method for the characterization of a fluorescently labelled compound in solution by analysing alterations in the fluorescence spectrum of the fluorescently labelled compound in combination with a defined temperature perturbation. An example in this context is microscale thermophoresis as known in the art and described in, e.g., WO 2008/061706.

In a specific embodiment of the present invention, alterations in the fluorescence spectrum of said ternary complex are analysed by microscale thermophoresis, temperature-related intensity change or spectral shift technology. Microscale thermophoresis (MST) refers to a technique for measuring biomolecular interactions - an MST instrument detects the motion of fluorescent molecules along a microscopic temperature gradient, which reflects changes in the molecular hydration shell, charge, or size. Temperature-Related Intensity Change (TRIC) refers to techniques that build upon the principle that the chemical environment around a fluorophore bound to a target molecule changes when the target molecule interacts with a ligand, causing a variation in the intensity of the fluorescence. During TRIC measurements a brief and precise laser-induced temperature increase is applied to amplify the change in fluorescence intensity which is related to the amount of ligand bound in the ligand-receptor interaction to be quantified. Spectral shift is a fluorescence-based biophysical technique used for quantifying the strength of molecular interactions. Spectral Shift builds upon the principle that the chemical environment around a fluorophore bound to a target molecule changes when the target molecule interacts with a ligand, causing a subtle wavelength shift in the emission spectrum of the fluorophore in isothermal conditions. During a Spectral Shift measurement, a ratiometric measurement is reported by recording the emission fluorescence simultaneously at two preselected wavelengths.

In context with the present invention, means for exciting may be an excitation light source, for example at least one light source from the group consisting of laser, laser fibre laser, diode laser, LED, HXP, Halogen, LED-Array, and HBO.

In a preferred embodiment of the present invention, where spectral shift technology is employed as described herein, a "dual-emission" configuration is used in combination with "red" fluorescent labels, such as Cy5, RFP, and other dyes known in the art and described herein. Since such labels have their excitation maximum at approx. 650 nm with a secondary excitation peak at approx. 600 nm and the emission maximum at approx. 660 nm, well-suited excitation and emission wavelengths include excitation between approx. 570 nm and 615 nm, detection of the first emission between approx. 625 nm and 650 nm and detection of the second emission between approx. 670 nm and 725 nm. The person skilled in the art is aware that in the context of the present invention an emission maximum can be a local emission maximum or an absolute emission maximum. Alternatively, the detection can be around a saddle point of the emission spectrum instead of an emission maximum. Small changes (e.g., wavelength shifts) in the regions flanking the emission maximum have a relatively large impact on the alterations of a fluorescence spectrum (e.g., in terms of intensities).

According to the present invention, preferable means for detecting the excited fluorescently labeled compound and its interaction with the fluorescence quencher, particularly for detecting the fluorescence, may be any suitable device selected from the group consisting of charge coupled device (CCD) cameras (2D or line-scan CCD), Line-Cameras, Photomultiplier Tubes (PMT), silicon photomultipliers (siPMs), avalanche photodiodes (APD), photodiode arrays (PDAs), complementary metal-oxide-semiconductor (CMOS) cameras and the like.

In context with the present invention, means for detecting may be a light detector, for example at least one detector from the group consisting of PMT, siPM, APD, CCD, and CMOS camera.

In a preferred embodiment of the method of the present invention for quantifying the formation of a complex composed of a first, second and third compound, wherein said second compound is capable of attaching to said first and third compound, comprising equipping said first or third compound with a fluorescent quencher, while the other one of the two is equipped with a fluorescent label, the second compound is absent.

Accordingly, in said preferred embodiment, the first and third compound is present.

Accordingly, the present invention provides for a method for quantifying the formation of preferably a binary complex composed of a first and third compound, comprising equipping said first or third compound with a fluorescent quencher, while the other one of the two is equipped with a fluorescent label. Preferably, said first and third compounds are proteins.

As described herein, either said first or third compound is equipped with a fluorescence quencher, while the other one of the two is equipped with a fluorescent label. Accordingly, if the fluorescent label and the fluorescent quencher are in proximity such that the fluorescent label and fluorescent quencher can, so to say, interact, due to formation of a complex between said first and third compound, the fluorescence may decrease. Said fluorescence originates from the fluorescent label.

Of course, if the fluorescent label and the fluorescent quencher are not in such proximity, fluorescence may not decrease.

That said, it is preferred that the formation of a complex between said first and third compound results in a decrease of fluorescence. This is because, as explained before, the formation of a complex between the first and third compound brings together a fluorescent label and a fluorescent quencher, thereby decreasing fluorescence (originating from the fluorescent label of either the first or third compound).

The decrease in fluorescence may be used as an indicator of the dimerization or even multimerization of the first and third compound. Accordingly, for the preferred embodiment of the method of the present invention, wherein the second compound is absent, it is preferred that a decrease of fluorescence is indicative of either desired or undesired complex formation, e.g. dimerization or multimerization.

The skilled person will understand whether or not complex formation between said first and third compound is desired. For example, by utilizing the preferred embodiment of the present invention, the skilled person may test two compounds, e.g. proteins for dimerization or even multimerization. Such dimerization or multimerization may be desired, e.g. for testing whether or not two proteins dimerize or even multimerize; or may be undesired, e.g. for investigating two proteins for their tendency to aggregate by dimerization or even multimerization.

Accordingly, the present invention provides for a method for determining whether or not a first and third compound form a binary complex, comprising equipping said first or third compound with a fluorescent quencher, while the other one of the two, i.e. the other one of the first or third compound, is equipped with a fluorescent label, wherein a decrease of fluorescence is indicative of either desired or undesired complex formation, in particular dimerization. Likewise, no decrease of fluorescence may be indicative of no desired or undesired complex formation, in particular dimerization.

The present invention also provides for the use of a fluorescence quencher for quantifying complex formation of a first and third compound, wherein said first or third compound is equipped with a fluorescence quencher, while the other one of the two, i.e. the other one of the first or third compound, is equipped with a fluorescent label. Preferably, said complex is a binary complex.

The present invention also provides the use of a fluorescence quencher for quantifying ternary complex formation of a first, second and third compound, wherein said second compound is capable of attaching to said first and third compound, wherein said first or third compound is equipped with a fluorescence quencher, while the other one of the two is equipped with a fluorescent label.

Accordingly, in one embodiment of the present invention, and by way of a non-limiting example, in which alterations in fluorescence emissions are being used to quantify the formation of a ternary complex comprising a target protein, a PROTAC, and an E3 ubiquitin ligase, in which the target protein is equipped with a fluorescent dye and the E3 ubiquitin ligase is equipped with a fluorescence quencher, the recording of fluorescence emission intensity may comprise the following steps:
(a) providing a sample with said fluorescently labelled target protein, a PROTAC with appropriate ligand domains, and an E3 ubiquitin ligase labelled with the fluorescence quencher in a solution;
(b) exciting the labelled target protein and detecting the fluorescence emission intensity of the excited protein;
(c) repeating the first two steps multiple times at incrementally increasing concentrations of PROTAC;
(d) quantifying the formation of ternary complex formed between the three compounds as described in step (a) through the anticipated decrease in fluorescence intensity that shall occur upon interaction between the fluorescent dye and the fluorescence quencher that occurs upon ternary complex formation;
(e) using the resultant fluorescence emission recordings to identify key biophysical properties of the interaction between the three compounds, such as the binary K_{d}s and Rₘₐₓ.

The present invention also relates to the use of a kit for performing the methods as described and provided herein in context with the present invention, said kit comprising (i) a fluorescence quencher ready to be coupled to said first or third compound of a ternary complex, i.e. a fluorescence quencher that can be coupled to said first or third compound of a ternary complex, (ii) a fluorescent label ready to be coupled to said first or third compound of a ternary complex, i.e. a fluorescence label that can be coupled to said first or third compound of a ternary complex, wherein said first compound or third compound is a component of an ubiquitin protein ligase complex. The kit preferably further comprises an instruction manual explaining the use of the kit in at least one of the methods of the present invention. Furthermore, in context with the kit for use in performing the method as described and provided herein, said fluorescent quenchers and fluorescent labels may preferably be as defined herein in context with one of the methods of the present invention.

The present invention also relates to the use of the system as described and provided herein, said system comprising (i) at least a first and third target protein, (ii) a fluorescence quencher ready to be coupled to one of the two target proteins, i.e. a fluorescence quencher that can be coupled to one of the two target proteins, (iii) a fluorescent label ready to be coupled to the other of the two target proteins, i.e. a fluorescent label that can be coupled to the other of the two target proteins, (iv) a second compound is capable of attaching to said first and third compound and (v) a device configured to analyse alterations in the fluorescence spectrum upon ternary complex formation between said first, second and third compound, wherein said first compound is as per any of the embodiments described herein, wherein said second compound is as defined as per any of the embodiments described herein, wherein said third compound is as defined as per any of the embodiments described herein, where said device is configured to perform microscale thermophoresis, temperature-related intensity change or spectral shift measurement.
The present invention can also be characterized by the following items:
1. A method for quantifying the formation of a complex composed of a first, second and third compound, wherein said second compound is capable of attaching to said first and third compound, comprising equipping said first or third compound with a fluorescent quencher, while the other one of the two is equipped with a fluorescent label.
2. A method for screening whether a second compound is capable of attaching to a first and third compound to form a complex, comprising detecting whether a complex is formed between said first, second and third compound, wherein said first or third compound is equipped with a fluorescent quencher, while the other one of the two is equipped with a fluorescent label.
3. The method of any one of the preceding items, wherein said first and said third compounds are proteins.
4. The method of any one of the preceding items, wherein said second compound is a compound with a molecular weight less than 2000 Da.
5. The method of any one of the preceding items, wherein said second compound comprises (i) a target protein binding moiety and (ii) a ubiquitin pathway protein binding moiety.
6. The method of item 5, wherein (i) said target protein binding moiety is covalently coupled to (ii) said ubiquitin pathway protein binding moiety.
7. The method of any one of items 5 to 6, wherein said (i) target protein binding moiety is capable of attaching to a target protein which is a first compound as defined in any one of the preceding items.
8. The method of any one of items 5 to 7, wherein said (ii) ubiquitin pathway protein binding moiety is capable of attaching to a ubiquitin pathway protein which is a third compound as defined in any one of the preceding items.
9. The method of any one of items 5 to 8, wherein said (ii) ubiquitin pathway protein binding moiety is a ligand for a component of a ubiquitin protein ligase complex.
10. The method of any one of items 5 to 9, wherein said ubiquitin pathway protein effects ubiquitination of said target protein.
11. The method of any one of items 5 to 10, wherein said ubiquitin pathway protein is a component of a ubiquitin protein ligase complex.
12. The method of item 11, wherein said component of a ubiquitin protein ligase complex is a ubiquitin protein ligase.
13. The method of item 12, wherein the ubiquitin protein ligase is an E3 ubiquitin ligase.
14. The method of any one of the preceding items, wherein said second compound is a proteolysis targeting chimera (PROTAC).
15. The method of any one of items 1 to 3, wherein said second compound is a bifunctional polypeptide, wherein said bifunctional polypeptide is preferably a bispecific antibody.
16. The method of any one of the preceding items, wherein said third compound is a component of a ubiquitin protein ligase complex, wherein preferably said component of a ubiquitin protein ligase complex is a ubiquitin protein ligase.
17. The method of any one of the preceding items, wherein the fluorescent label is a fluorescent dye.
18. The method of item 17, wherein said fluorescent dye is Cy5, Cy3, Atto647, Atto647N, Alexa647, Dy647, or variants thereof.
19. The method of any one of the preceding items, wherein the fluorescence quencher is a black hole quencher^{®} is BHQ1, BHQ2 or BHQ3.6
20. The method of any one of the preceding items, wherein formation of the complex comprises analysing alterations in the fluorescence spectrum upon complex formation between said first, second and third compound.
21. The method of item 20, wherein alterations in the fluorescence spectrum are alterations in the fluorescence emission spectrum.
22. The method of any one of items 20 to 21, wherein an alteration in the fluorescence spectrum is indicative of complex formation.
23. The method of any one of items 20 to 22, wherein the alterations in the fluorescence spectrum of said complex are analysed by microscale thermophoresis, temperature-related intensity change or spectral shift technology.
24. Use of a fluorescence quencher for quantifying complex formation of a first, second and third compound, wherein said second compound is capable of attaching to said first and third compound, wherein said first or third compound is equipped with a fluorescence quencher, while the other one of the two is equipped with a fluorescent label.
25. Use of a kit for performing the method of any one of items 1 to 23, said kit comprising (i) a fluorescence quencher ready to be coupled to said first or third compound of a complex.
26. The use of item 25, said kit further comprising (ii) a fluorescent label ready to be coupled to said first or third compound of a complex.
27. The use of item 25 or 26, wherein said first compound or third compound is a component of a ubiquitin protein ligase complex.
28. A kit for use in the method of any one of items 1 to 23, said kit comprising (i) a fluorescence quencher ready to be coupled to said first or third compound of a complex.
29. The kit for the use of item 28, said kit further comprising (ii) a fluorescent label ready to be coupled to said first or third compound of a complex.
30. A system comprising (i) at least a first and third target protein, (ii) a fluorescence quencher ready to be coupled to one of the two target proteins, (iii) a fluorescent label ready to be coupled to the other of the two target proteins, (iv) a second compound is capable of attaching to said first and third compound and (iv) a device configured to analyse alterations in the fluorescence spectrum upon complex formation between said first, second and third compound.
31. The system of item 30, wherein said first compound is as defined in any one of items item 1 and 2.
32. The system of item 30 or 31, wherein said second compound is as defined in any one of items 1 to 15.
33. The system of any one of items 30 to 32, wherein said third compound is as defined in any one of items 1, 2 and 16.
34. The system of any one of items 30 to 33, where said device is configured to perform microscale thermophoresis, temperature-related intensity change or spectral shift measurement.
35. Use of the system of any one of items 30 to 34 for detecting complex formation.
36. The use of item 35, wherein said detection of complex formation is pursuant to the method of any one of items 1 to 23.
37. The method of any one of items 1 and 2 to 23, wherein said second compound is absent.
38. The method of item 37, wherein formation of a complex results in a decrease of fluorescence.
39. The method of item 38, wherein a decrease of fluorescence is indicative of (un)desired complex formation.
40. A method for determining whether or not a first and third compound form a binary complex, comprising equipping said first or third compound with a fluorescent quencher, while the other one of the two is equipped with a fluorescent label, wherein a decrease of fluorescence is indicative of either desired or undesired complex formation, in particular dimerization.
41. Use of a fluorescence quencher for quantifying complex formation of a first and third compound, wherein said first or third compound is equipped with a fluorescence quencher, while the other one of the two is equipped with a fluorescent label.
42. The use of item 41, wherein said complex is a binary complex.

The embodiments which characterize the present invention are described herein, shown in the Figures, illustrated in the Examples, and reflected in the claims.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% or 2% of a given value or range, and also comprise the respective exact numeric value.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims.

A better understanding of the present invention and of its advantages will be had from the following examples, offered for illustrative purposes only. The examples are not intended to limit the scope of the present invention in any way. The term "example(s)" and "figure(s)" are used interchangeably in this document.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

### IV. BRIEF DESCRIPTION OF THE FIGURES

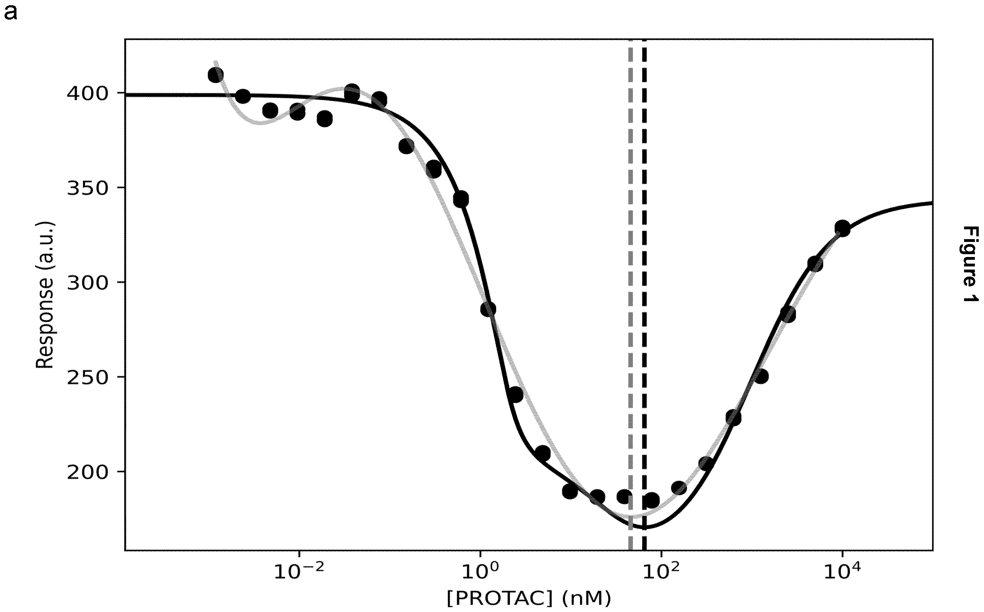

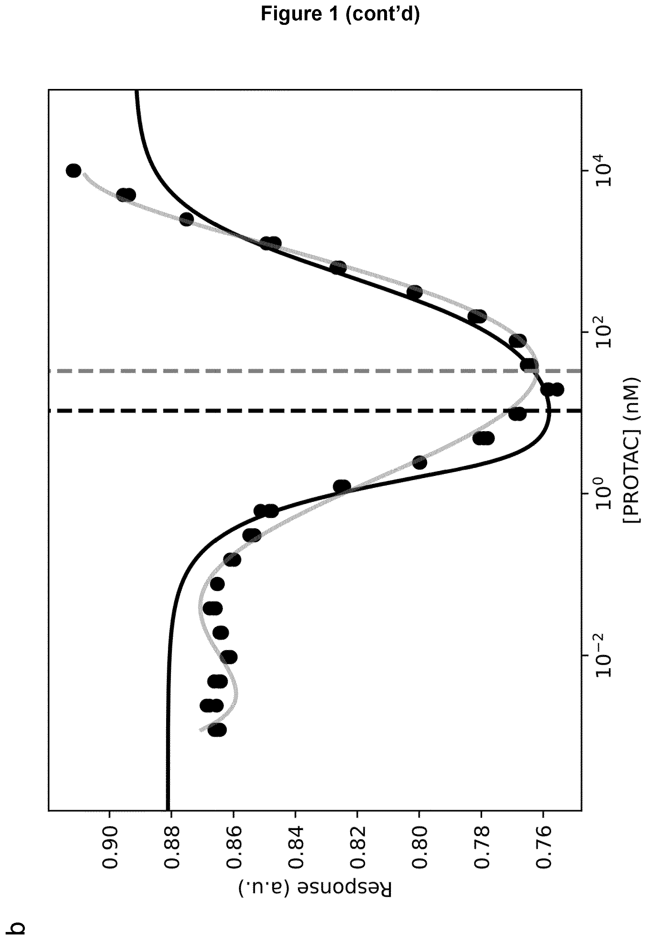

Figure 1 Analysis of ternary complex formation via quantification of fluorescence quenching

### V. EXAMPLES

Ternary complex formation was evaluated using the method of the present invention, for a complex comprising the PROTAC MZ1, the VCB ubiquitin ligase complex, and the target protein bromodomain-containing protein 2 (BRD2).

### Materials and Methods

Assays were performed in a solution comprised of the following - 50mM Tris-HCl, pH 7.8, 150mM NaCl, 10mM MgCl₂, 2mM GSH, 0.01 % Pluronic F-127, 1% DMSO. BRD2 was labelled with the RED-NHS 2nd Generation Dye (NanoTemper Technologies GmbH, hereafter NTT) in carbonate buffer pH 8.3 in 1:3 protein to dye ratio following the manufacturer's protocol, whilst the VCB complex was conjugated with the fluorescence quencher Tide Quencher 5WS Maleimide (AAT Bioquest) in phosphate buffer pH 7 in 1:3 protein to quencher ratio, with free dye removed by desalting with a PD-10 column. Assays were performed with BRD2 at a fixed concentration of 5nM and VCB at a fixed concentration of 25nM. MZ1 PROTAC was applied at 24 incrementally increasing concentrations from 1pM to 10µM. Fluorescence measurements were taken at each concentration. Samples were loaded into Premium Capillaries (NTT) and placed into the sample tray of a Monolith X instrument (NTT) excited at 593 +/- 23 nm and emission recorded at 640 +/- 10 and 697 +/- 58 nm. Fluorescence emission was recorded for 1 second and averaged. The ratio value is calculated by dividing the 670 nm emission by the 650 nm. Both the raw fluorescence emission at 650 nm and the ratio value is then plotted as a function of linker concentration.

Fitting was performed using the ternary model described in Douglass et al. (2013, doi.org/10.1021/ja311795d). As described in Douglass et al., direct calculation of the ternary complex concentration as a function of PROTAC concentration is intractable. However, it is possible to calculate the opposite relationship, the expected PROTAC concentration as a function of ternary complex concentration, as well as the PROTAC concentration which maximizes ternary complex formation. Using these relations we generated a dense grid of logarithmically spaced ternary complex concentrations and their corresponding PROTAC concentrations, which is our model output. We then did a nonlinear fit of the cooperativity parameter and the initial and final saturation values (in photon counts). Nonlinear fitting was performed using the curve_fit routine from the optimize package in SciPy with Python distribution 3.10.8. Numerical optimization in curve_fit was performed using the Trust Region Reflective ('trf') algorithm with a step size of 1×10⁻² and a non-negative lower bound on all variables. In order to compare the model estimate of the PROTAC concentration which maximizes ternary complex formation to the apparent maximal ternary complex concentration from the data, we fitted a 6^{th} order polynomial to the data to obtain a smooth estimate of the data, where the order of polynomial was chosen using the Bayesian Information Criterion. We took the concentration at the minimum fluorescence response (maximal quenching) of the smoothed data approximation as the point of maximal ternary complex formation. Both the Douglass model fit and the smoothed data approximation gave similar results.

### Example 1

**Figure 1****,** panel **a** shows the raw fluorescence intensity changes across the applied MZ1 PROTAC concentrations. A ternary fitting model (black line) estimated binding cooperativity (α) at 39.6, with peak ternary binding ([3°] max) as 64.8nM. 6th order polynomial fitting (grey line) estimated [3°] max as 45.6nM.

This demonstrates the characteristic response curve anticipated when employing the methods of the present invention. As PROTAC concentration increased, fluorescence intensity initially decreases as ternary complex formation brings the fluorescent label and fluorescent quencher into proximity. A peak in ternary complex formation corresponds to the lowest fluorescence intensity. As concentration of PROTAC increases further, the hook effect can be clearly seen from the subsequent increase in detected fluorescence intensity, indicative of a decrease in ternary complex formation.

**Figure 1**, panel **b** shows the spectral shift in fluorescence emission ratio between 670nm and 650nm emission wavelengths (670nm/650nm), for the same range of applied concentrations of MZ1 PROTAC as shown in panel a. The ternary fitting model (black line) estimates α to be 21.2 and [3°]max to be 10.7 nM. The 6th order polynomial fitting (grey line) estimates [3°]max to be 33.1 nM.

### References

1. Sakamoto et al., Protacs: chimeric molecules that target proteins to the Skp1-Cullin-F box complex for ubiquitination and degradation, PNAS, 2001; Jul 17;98(15):8554-9. doi: 10.1073/pnas.141230798. PubMed PMID: 11438690
2. Cecchini et al., From Conception to Development: Investigating PROTACs Features for Improved Cell Permeability and Successful Protein Degradation, Frontiers in Chemistry, 2021; Apr 20:9:672267. doi: 10.3389/fchem.2021.672267, PubMed PMID: 33959589
3. Mak et al., Kinetic analysis of ternary and binary binding modes of the bispecific antibody emicizumab, mABs, 2023; Jan-Dec;15(1): 2149053. doi:10.1080/19420862.2022.2149053., PubMed PMID: 36453702
4. Chen et al., Development of a mechanism of action-reflective, dual target cell-based reporter bioassay for a bispecific monoclonal antibody targeting human CTLA-4 and PD-1, mAbs, 2021, 13(1): 1914359, doi: 10.1080/19420862.2021.1914359, PubMed PMID: 33870864
5. Xie et al., Elucidation of genome-wide understudied proteins targeted by PROTAC-induced degradation using interpretable machine learning, PLoS Computational Biology, 2023, 19(8): e1010974, doi: 10.1371/journal.pcbi.1010974, Pubmed PMID: 37590332
6. Burslem et al., The Advantages of Targeted Protein Degradation over Inhibition: a RTK Case Study, Cell Chemical Biology, 2018, Jan 18; 25(1): 67-77.e3., doi: 10.1016/j.chembiol.2017.09.009, PubMed PMID: 29129716
7. Nowak et al., Plasticity in binding confers selectivity in ligand-induced protein degradation, Nature Chemical Biology, 2018, Jul;14(7):706-714. doi: 10.1038/s41589-018-0055-y, PubMed PMID: 29892083
8. Han, B., A suite of mathematical solutions to describe ternary complex formation and their application to targeted protein degradation by heterobifunctional ligands, Journal of Biological Chemistry, 2020, Nov 6;295(45):15280-15291. doi: 10.1074/jbc.RA120.014715, PubMed PMID: 32859748
9. Bai et al., Rationalizing PROTAC-Mediated Ternary Complex Formation Using Rosetta, Journal of Chemical and Informatical Modelling, 2021, Mar 22;61(3):1368-1382. doi: 10.1021/acs.jcim.0c01451, PubMed PMID: 33625214
10. Songsivilai et al., Bispecific antibody: a tool for diagnosis and treatment of disease, Clinical and Experimental Immunology, 1990, 79(3): 315-321., doi: 10.1111/j.1365-2249.1990.tb08089.x, PubMed PMID: 2180597
11. Kostelny et al., Formation of a bispecific antibody by the use of leucine zippers, Journal of Immunology, 1992, Mar 1;148(5):1547-53., doi.org/10.4049/jimmunol.148.5.1547, PubMed PMID: PMID: 1531669
12. Jones et al., Replacing the complementarity-determining regions in a human antibody with those from a mouse, Nature, 1986, 321(6069):522-5. doi: 10.1038/321522a0., PubMed PMID: 3713831
13. Reichmann et al., Reshaping human antibodies for therapy, Nature, 1988, Mar 24;332(6162):323-7. doi: 10.1038/332323a0, PubMed PMID: 3127726
14. Presta, L., Antibody Engineering, Current Opinions in Biotechnology, 1992, Aug;3(4):394-8. doi: 10.1016/0958-1669(92)90168-1, PubMed PMID: 1368441
15. Lambert, TJ., FPbase: a community-editable fluorescent protein database., Nature Methods., 2019, 16, 277-278. doi:10.1038/s41592-019-0352-8, PubMed PMID: 30886412
16. Klymchenko, AS., Solvatochromic and Fluorogenic Dyes as Environment-Sensitive Probes: Design and Biological Applications, Accounts of Chemical Research, 2017, Feb 21;50(2):366-375. doi: 10.1021/acs.accounts.6b00517, PubMed PMID: 28067047
17. Douglass et al., A comprehensive mathematical model for three-body binding equilibria, Journal of the American Chemical Society, 2013, Apr 24;135(16):6092-9. doi: 10.1021/ja311795d, PubMed PMID: 23544844

## Claims

1. A method for quantifying the formation of a complex composed of a first, second and third compound, wherein said second compound is capable of attaching to said first and third compound, wherein said first or third compound is equipped with a fluorescence quencher, while the other one of the first and third compound is equipped with a fluorescent label, comprising generating a concentration-response curve that shows the peak of ternary complex formation, whereby said peak corresponds to the lowest level of fluorescence emission intensity, and determining whether subsequent to said lowest level of fluorescence emission intensity of fluorescence increases in said concentration-response curve, which is indicative of a hook effect, thereby quantifying ternary complex formation.

2. A method for screening whether a second compound is capable of attaching to a first and third compound to form a complex, comprising detecting whether a complex is formed between said first, second and third compound, wherein said first or third compound is equipped with a fluorescence quencher, while the other one of the first and third compound is equipped with a fluorescent label, comprising generating a concentration-response curve that shows the peak of ternary complex formation, whereby said peak corresponds to the lowest level of fluorescence emission intensity, and determining whether subsequent to said lowest level of fluorescence emission intensity of fluorescence increases in said concentration-response curve, which is indicative of a hook effect, thereby screening a second compound capable of attaching to the first and third compound.

3. The method of any one of the preceding claims, wherein said first compound and said third compound are proteins.

4. The method of any one of the preceding claims, wherein said second compound comprises (i) a target protein binding moiety that is capable of attaching to a target protein which is a first compound as defined in any one of the preceding claims and (ii) an ubiquitin pathway protein binding moiety that is capable of attaching to a ubiquitin pathway protein, wherein the ubiquitin pathway protein effects ubiquitination of the target protein, which is a third compound as defined in any one of the preceding claims, and (iii) a linker moiety that covalently couples them.

5. The method of claim 4, wherein said (ii) ubiquitin pathway protein binding moiety is a ligand for a component of an ubiquitin protein ligase complex, wherein said ubiquitin pathway protein is preferably a component of an ubiquitin protein ligase complex, wherein said component of an ubiquitin protein ligase complex is more preferably an ubiquitin protein ligase.

6. The method of any one of the preceding claims, wherein said second compound is a proteolysis targeting chimera (PROTAC).

7. The method of any one of the preceding claims, wherein said third compound is a component of an ubiquitin protein ligase complex, wherein said component of the ubiquitin protein ligase complex effects ubiquitination of the target protein, wherein preferably said component of an ubiquitin protein ligase complex is a ubiquitin protein ligase.

8. The method of any one of claims 1 to 3, wherein said second compound is a bifunctional polypeptide, wherein said bifunctional polypeptide is preferably a bispecific antibody.

9. The method of any one of the preceding claims, wherein the fluorescent label is a fluorescent dye, wherein said fluorescent dye is preferably Cy5, Cy3, Atto647, Atto647N, Alexa647, Dy647, or variants thereof.

10. The method of any one of the preceding claims, wherein the fluorescence quencher is a black hole quencher^{®}, for example BHQ1, BHQ2 or BHQ3.6

11. The method of any one of the preceding claims, wherein quantifying or screening for the formation of the complex comprises analysing alterations in the fluorescence spectrum that occur upon complex formation between said first, second and third compound, wherein preferably alterations in the fluorescence spectrum alterations in the fluorescence emission spectrum, wherein more preferably an alteration in the fluorescence spectrum is indicative of complex formation.

12. The method of claim 11, wherein alterations in the fluorescence spectrum of said complex are analyzed by microscale thermophoresis, temperature-related intensity charge or spectral shift technology.

13. Use of a fluorescence quencher for quantifying complex formation of a first, second and third compound, wherein said second compound is capable of attaching to said first and third compound, wherein said first or third compound is equipped with a fluorescence quencher, while the other one of the first and third compound is equipped with a fluorescent label, wherein said use is in a method of any one of claims 1 to 12.

14. Use of a kit for performing the method of any one of claims 1 to 12, said kit comprising (i) a fluorescence quencher that can be coupled to said first or third compound of a complex, (ii) a fluorescent label that can be coupled to said first or third compound of a complex, preferably wherein said first compound or third compound is a component of an ubiquitin protein ligase complex, wherein said component of the ubiquitin protein ligase complex effects ubiquitination of a target protein.

15. A system comprising (i) at least a first and third target protein, (ii) a fluorescence quencher that can be coupled to one of the two target proteins, (iii) a fluorescent label that can be coupled to the other of the two target proteins, (iv) a second compound is capable of attaching to said first and third compound and (v) a device configured to analyse alterations in the fluorescence spectrum upon complex formation between said first, second and third compound, wherein said first compound is as defined in any one of claims 1, 2, and 3, wherein said second compound is as defined in any one of claims 1 to 8, wherein said third compound is as defined in any one of claims 1, 2, 3, and 7, where said device is configured to perform microscale thermophoresis, temperature-related intensity change or spectral shift measurement, wherein the analysis of alterations in the fluorescence spectrum is pursuant to the method of any one of claims 1 to 12.
